# EUROPÄISCHE PATENTSCHRIFT

(11) **EP 1 244 419 B1**
(45) Veröffentlichungstag und Bekanntmachung des Hinweises auf die Patenterteilung: **09.11.2005**
(21) Anmeldenummer: 00991207.2
(22) Anmeldetag: 18.12.2000
(51) Int. Cl.: A61K 7/13

(54) **MITTEL ZUM FÄRBEN VON KERATINHALTIGEN FASERN**
AGENT FOR DYEING KERATIN CONTAINING FIBERS
AGENT POUR COLORER DES FIBRES KERATINIQUES

(30) Priorität: 24.12.1999 DE 19962873; 13.12.2000 DE 10061990
(43) Veröffentlichungstag der Anmeldung: 02.10.2002
(73) Patentinhaber: Henkel Kommanditgesellschaft auf Aktien, 40589 Düsseldorf (DE)
(72) Erfinder: ROSE, David, 40723 Hilden (DE); HÖFFKES, Horst, 40595 Düsseldorf (DE); MEINIGKE, Bernd, 51381 Leverkusen (DE)
(86) Internationale Anmeldenummer: PCT/EP2000/012893
(87) Internationale Veröffentlichungsnummer: WO 2001/047477

(56) Entgegenhaltungen:
- EP-A- 0 013 257
- EP-A- 0 873 746
- EP-A- 1 166 753
- DE-A- 19 732 016
- GB-A- 1 412 149
- US-A- 3 749 714
- DATABASE WPI Derwent Publications Ltd., London, GB; AN 1989-319875 XP002169922 & JP 01 237452 A (SAMPO KAGAKU KENKYUSHO KK) 21. September 1989 (1989-09-21)

## Beschreibung

Die Erfindung betrifft ein Mittel zum Färben von keratinhaltigen Fasern, insbesondere menschlichen Haaren, das spezielle färbende Komponente enthält, die Verwendung dieser speziellen Komponenten als färbende Komponente in Haarfärbemitteln sowie ein Verfahren zum Färben von keratinhaltigen Fasern, insbesondere menschlichen Haaren.

Für das Färben von keratinhaltigen Fasern, beispielsweise Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2'-hydroxyethyl)-p-phenylendiamin, 2-(2',5'-Diaminophenyl)-ethanol, 2-(2',5'-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2'-hydroxyethylamino)-anisol, 1-Phenyl-3-methylpyrazolon-5, 2,4-Dichlor-3-aminophenol, 1,3-Bis-(2',4'-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie beispielsweise H₂O₂, was in einigen Fällen Schädigungen der Faser zur Folge haben kann. Desweiteren können einige Oxidationsfarbstoffvorprodukte beziehungsweise bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung der unten näher beschriebenen speziellen Komponenten zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie zum Beispiel H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel 1 dargestellten Verbindungen sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agenzien soll dabei jedoch nicht prinzipiell ausgeschlossen werden.

Gegenstand der Erfindung ist die Verwendung eines Mittels zum Färben von keratinhaltigen Fasern, insbesondere menschlichen Haaren, enthaltend als färbende Komponente Verbindungen mit der Formel I,

A-CH=B-CH=A' (I)

in der =B- steht für eine Gruppe =CR¹- oder eine Gruppe 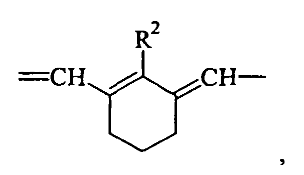 A ist ausgewählt aus den Gruppen 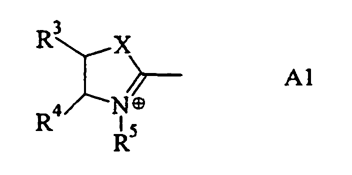 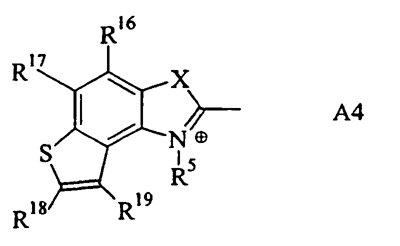 und A' ist ausgewählt aus den Gruppen 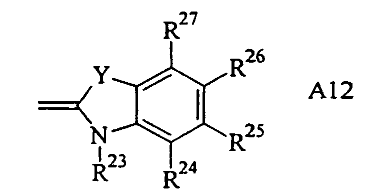 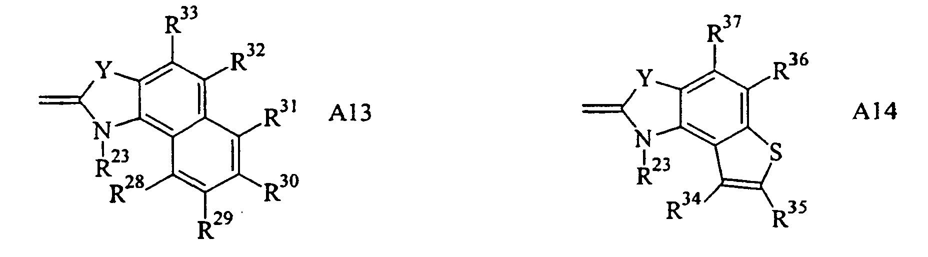 wobei
- X und Y: stehen jeweils für ein Schwefelatom,
- R¹: sieht für Wasserstoff oder eine Gruppe NH₂,
- R²: steht für Wasserstoff oder ein Halogenatom, insbesondere ein Chloratom,

- R⁵ und R²³: stehen unabhängig voneinander für eine C₁- bis C₄-Alkylgruppe, die gegebenenfalls mit einer oder mehreren Hydroxygruppe(n) oder einer Säure- oder Säureaniongruppe, insbesondere einer SO₃⁻-Gruppe substituiert ist,
- R¹⁷, R²⁵ und R²⁶: stehen unabhängig voneinander für Wasserstoff eine C₁- bis C₄-Alkylgruppe, eine C₁- bis C₄-Alkoxygruppe, eine Phenylgruppe, eine SO₂CF₃-Gruppe oder eine mit einer C₁- bis C₄-Alkylgruppe veresterte Carboxygruppe, und
die übrigen Reste stehen unabhängig voneinander für Wasserstoff, ein Halogenatom, eine C₁- bis C₄-Alkylgruppe oder eine C₁- bis C₄-Alkoxygruppe, die gegebenenfalls durch eine oder mehrere Hydroxygruppen oder Halogenatome substituiert sind,
wobei die positive Ladung durch eine negative Ladung in einem der Reste oder durch ein Säureanion ausgeglichen werden kann.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie beispielsweise Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose und synthetischer Fasern, wie beispielsweise Polyamid-, Polyacrylnitril-, Polyurethan- und Polyesterfasern verwendet werden.

Beispiele für die als Substituenten in den erfindungsgemäßen Verbindungen genannten, C₁- bis C₄-Alkylgruppen sind die Gruppen Methyl, Ethyl, Propyl, Isopropyl und Butyl. Ethyl und Methyl sind bevorzugte Alkylgruppen. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab.

Erfindungsgemäß besonders bevorzugt sind Verbindungen der Formel I, bei denen R⁵ und R²³ unabhängig voneinander stehen für eine Methylgruppe, eine Ethylgruppe, eine 2-Hydroxyethylgruppe oder eine 2-Sulfonatpropylgruppe.

Weiterhin besonders bevorzugt sind Verbindungen der Formel I, bei denen R¹⁷, R²⁵ und R²⁶ unabhängig voneinander stehen für Wasserstoff, eine Methylgruppe, eine Ethylgruppe, eine Methoxygruppe, eine Phenylgruppe, eine SO₂CF₃-Gruppe oder eine Carboxyethylgruppe.

In einer besonders bevorzugten Ausführungsform der vorliegenden Erfindung werden als Verbindungen der Formel I, eine oder mehrere der in Tabelle 1 genannten Verbindungen eingesetzt, wobei die in der Tabelle nicht definierten Substituenten für Wasserstoff stehen.

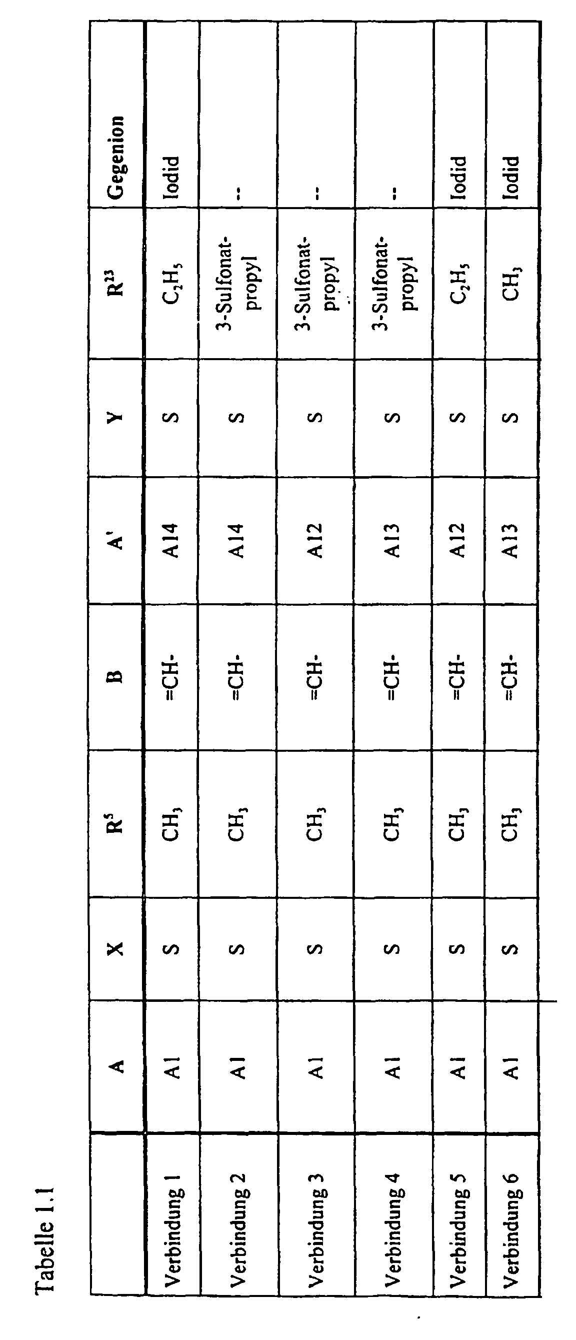

Als bevorzugte Säureanionen zum Ausgleich der positiven Ladung kommen Säureaniongruppe innerhalb der Verbindungen der Formel I selber oder freie Anionen, insbesondere Benzolsulfonat, p-Toluolsulfonat, Methansulfonat, Ethansulfonat, Propansulfonat, Perchlorat, Sulfat, Chlorid, Bromid, Jodid, Tetrachlorzinkat, Methylsulfate, Trifluormethansulfonat, Hexafluorphosphat und/oder Tetrafluorborat in Betracht.

Die Verbindungen mit der Formel I sind zum Teil literaturbekannt, im Handel erhältlich oder nach bekannten Syntheseverfahren herstellbar.

Die voranstehend genannten Verbindungen mit der Formel I werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet.

Die erfindungsgemäß eingesetzten Verbindungen mit der Formel I können als direktziehende Färbemittel oder in Gegenwart von üblichen Oxidationsfarbstoffvorprodukten, wie üblichen Kuppler- und Entwicklerkomponenten, die bereits im einleitenden Teil der Beschreibung genannt wurden, eingesetzt werden.

Färbemittel, die als einzige färbende Komponente die erfindungsgemäße Verbindungen enthalten, werden bevorzugt für Färbungen von gelb, orange bis hin zu rot eingesetzt.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazin, deren Derivaten sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Verbindungen mit der Formel I zum Einsatz kommen; ebenso können auch mehrere verschiedene Oxidationsfarbstoffvorprodukte und/oder Farbverstärker gemeinsam verwendet werden.

Auf die Anwesenheit von Oxidationsmitteln, wie beispielsweise H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, zum Beispiel aus der Gruppe der Nitrophenylendiamine, Nitroaminophenole, Anthrachinone oder Indophenole, wie beispielsweise die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel enthalten.

Es ist nicht erforderlich, daß die erfindungsgemäß enthaltenen Verbindungen der Formel I oder die fakultativ enthaltenen Oxidationsfarbstoffvorprodukte, Farbverstärker und direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, beispielsweise toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind zum Beispiel Cremes, Emulsionen, Gele oder auch tensidhaltige schäumende Lösungen wie zum Beispiel Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Citronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe beispielsweise eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, beispielsweise Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80).
Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengt er Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, beispielsweise Methylcellulose, Hydroxyalkylcellulose und Carboxymethylcellulose, Stärke-Fraktionen und Derivate wie Amylose, Amylopektin und Dextrine, Tone wie beispielsweise Bentonit oder vollsynthetische Hydrokolloide wie beispielsweise Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; beispielsweise werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind beispielsweise Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten. Der pH-Wert der gebrauchsfertigen Färbezubereitungen liegt üblicherweise zwischen 2 und 12, vorzugsweise zwischen 4 und 10.

Ein zweiter Gegenstand der vorliegenden Erfindung betrifft ein Verfahren zum Färben von keratinhaltigen Fasern, insbesondere menschlichen Haaren, worin ein Färbemittel, enthaltend mindestens eine Verbindung der Formel I sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Verbindungen der Formel I und die gegebenenfalls enthaltenen Oxidationsfarbstoffvorprodukte, Farbverstärker und direktziehenden Farbstoffe können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Verbindungen der Formel I und die gegebenenfalls enthaltenen Oxidationsfarbstoffvorprodukte, Farbverstärker und direktziehenden Farbstoffe können entweder getrennt oder zusammen gelagert werden, entweder in einer flüssigen bis pastösen Zubereitung (wäßrig oder wasserfrei) oder als trockenes Pulver. Bei der getrennten Lagerung werden die Komponenten erst unmittelbar vor der Anwendung innig miteinander vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Ausfärbungen

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol®techn.¹ | 4,0 |
| Texapon®N 28² | 40,0 |
| Dehyton®K³ | 25,0 |
| Eumulgin®B 2⁴ | 1,5 |
| destilliertes Wasser | 12,5 |

| | |
|---|---|
| ¹ C₁₂₋₁₈-Fettalkohol (Cognis) | |
| ² Natriumlaurylethersulfat (ca. 28 % Aktivsubstanz; CTFA-Bezeichnung: Sodium Laureth Sulfate) (Cognis) | |
| ³ Fettsäureamid-Derivat mit Betainstruktur der Formel R-CONH(CH₂)₃N⁺(CH₃)₂CH₂COO⁻ (ca. 30 % Aktivsubstanz; CTFA-Bezeichnung Cocoamidopropyl Betaine) (Cognis) | |
| ⁴ Cetylstearylalkohol mit ca. 20 Mol EO (CTFA-Bezeichnung: Ceteareth-20) (Cognis) | |

Auf Basis dieser Creme wurde dann folgende Haarfärbecremeemulsion hergestellt:

| | |
|---|---|
| Cremebasis | 50,0 |
| Färbekomponente | 7,5 mmol |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.

Die verwendeten Färbekomponenten sowie die Färbeergebnisse sind in der folgenden Tabelle dargestellt.

| **Färbekomponente** | **Farbton** |
|---|---|
| Verbindung 1 gemäß Tabelle 1 | Kalypsorot |
| Verbindung 2 gemäß Tabelle 1 | Melonenorange |
| Verbindung 3 gemäß Tabelle 1 | Zitronengelb |
| Verbindung 4 gemäß Tabelle 1 | Creme |
| Verbindung 5 gemäß Tabelle 1 | Rotorange |
| Verbindung 6 gemäß Tabelle 1 | Rot |

## Patentansprüche

1. Verwendung eines Mittels, enthaltend als färbende Komponente Verbindungen mit der Formel I,
A-CH=B-CH=A' (I)
in der =B- steht für eine Gruppe =CR¹- oder eine Gruppe 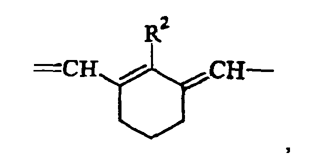 A ist ausgewählt aus den Gruppen 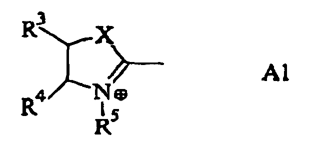 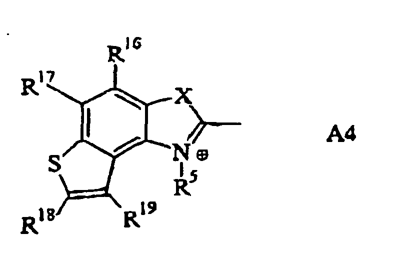 und A' ist ausgewählt aus den Gruppen 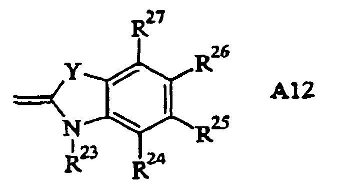 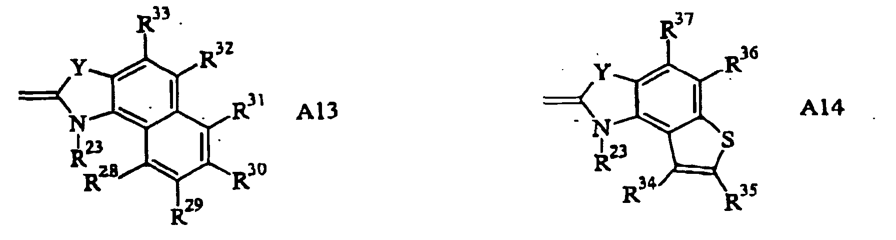 wobei
X und Y stehen jeweils für ein Schwefelatom,
R¹ steht für Wasserstoff, oder eine Gruppe NH₂,
R² steht für Wasserstoff oder ein Halogenatom, insbesondere ein Chloratom,
R⁵ und R²³ stehen unabhängig voneinander für eine C₁- bis C₄-Alkylgruppe, die gegebenenfalls mit einer oder mehreren Hydroxygruppe(n) oder einer Säure- oder Säureaniongruppe, insbesondere einer SO₃⁻-Gruppe substituiert ist,
R¹⁷, R²⁵ und R²⁶ stehen unabhängig voneinander für Wasserstoff, eine C₁- bis C₄-Alkylgruppe, eine C₁- bis C₄-Alkoxygruppe, eine Phenylgruppe, eine SO₂CF₃-Gruppe oder eine mit einer C₁- bis C₄-Alkylgruppe veresterte Carboxygruppe, und
die übrigen Reste stehen unabhängig voneinander für Wasserstoff, ein Halogenatom, eine C₁- bis C₄-Alkylgruppe oder eine C₁- bis C₄-Alkoxygruppe, die gegebenenfalls durch eine oder mehrere Hydroxygruppen oder Halogenatome substituiert sind,
wobei die positive Ladung durch eine negative Ladung in einem der Reste oder durch ein Säureanion ausgeglichen werden kann. zum Färben von keratinhaltigen Fasern, insbesondere menschlichen Haaren.

2. Verwendung nach Anspruch 1, **dadurch gekennzeichnet, daß** R⁵ und R²³ stehen unabhängig voneinander für eine Methylgruppe, eine Ethylgruppe, eine Hydroxyethylgruppe oder eine 2-Sulfonatpropylgruppe.

3. Verwendung nach einem der Ansprüche 1 oder 2, **dadurch gekennzeichnet, daß** R¹⁷, R²⁵ und R²⁶ stehen unabhängig voneinander für Wasserstoff, eine Methylgruppe, eine Ethylgruppe, eine Methoxygruppe, eine Phenylgruppe, eine SO₂CF₃-Gruppe oder eine Carboxyethylgruppe.

4. Verwendung nach einem der Ansprüche 1 bis 3, **dadurch gekennzeichnet, dass** als Verbindungen mit der Formel I eine der in Tabelle 1 genannten Verbindungen eingesetzt wird, wobei die in der Tabelle nicht definierten Substituenten für Wasserstoff stehen.
**Tabelle 1.1**
| | **A** | **X** | **R**^{**5**} | **B** | **A'** | **Y** | **R**^{**23**} | **Gegenion** |
|---|---|---|---|---|---|---|---|---|
| Verbindung 1 | A1 | S | CH₃ | =CH- | A14 | S | C₂H₅ | Iodid |
| Verbindung 2 | A1 | S | CH₃ | =CH- | A14 | S | 3-Sulfonat-propyl | -- |
| Verbindung 3 | A1 | S | CH₃ | =CH- | A12 | S | 3-Sulfonat-propyl | -- |
| Verbindung 4 | A1 | S | CH₃ | =CH- | A13 | S | 3-Sulfonat-propyl | -- |
| Verbindung 5 | A1 | S | CH₃ | =CH- | A12 | S | C₂H₅ | Iodid |
| Verbindung 6 | A1 | S | CH₃ | =CH- | A13 | S | CH₃ | Iodid |

5. Verwendung nach einem der Ansprüche 1 bis 4, **dadurch gekennzeichnet, dass** das Mittel Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

6. Verwendung nach einem der Ansprüche 1 bis 5, **dadurch gekennzeichnet, dass** das Mittel direktziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

7. Verwendung nach einem der Ansprüche 1 bis 6, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

8. Verwendung nach einem der Ansprüche 1 bis 7, **dadurch gekennzeichnet, daß** das Mittel Oxidationsmittel, insbesondere H₂O₂, in einer Menge von 0,01 bis 6 Gew.-%, bezogen auf die Anwendungslösung, enthält.

9. Verwendung nach einem der Ansprüche 1 bis 8, **dadurch gekennzeichnet, daß** das Mittel anionische, zwitterionische oder nichtionische Tenside enthält.

10. Verfahren zum Färben von keratinhaltigen Fasern, insbesondere menschlichen Haaren, worin ein Färbemittel, enthaltend mindestens eine Verbindung mit der Formel I sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Use of an agent comprising, as dyeing component, compounds with the formula I,
A-CH=B-CH=A' (I)
in which =B- is a group =CR¹- or a group 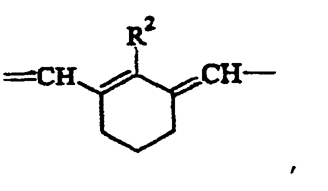 A is chosen from the groups 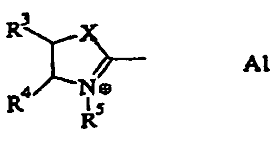 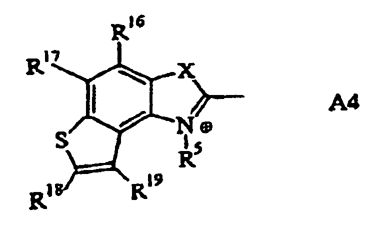 and A' is chosen from the groups 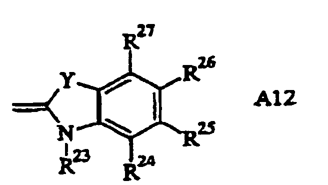 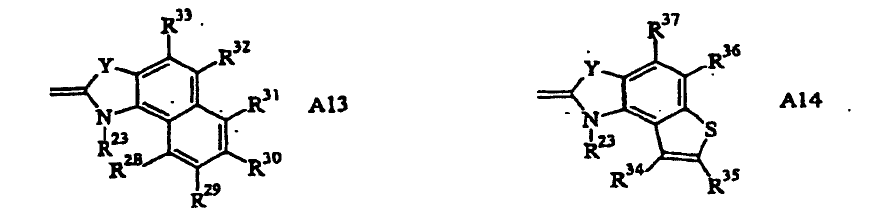 where
X and Y are in each case a sulphur atom,
R¹ is hydrogen or a group NH₂,
R² is hydrogen or a halogen atom, in particular a chlorine atom,
R⁵ and R²³, independently of one another, are a C₁-C₄-alkyl group, which is optionally substituted by one or more hydroxy group(s) or an acid or acid anion group, in particular an SO₃⁻ group,
R¹⁷, R²⁵ and R²⁶, independently of one another, are hydrogen, a C₁-C₄-alkyl group, a C₁-C₄-alkoxy group, a phenyl group, an SO₂CF₃ group or a carboxy group esterified with a C₁-C₄-alkyl group, and
the other radicals, independently of one another, are hydrogen, a halogen atom, a C₁-C₄-alkyl group or a C₁-C₄-alkoxy group, which are optionally substituted by one or more hydroxy groups or halogen atoms,
where the positive charge can be balanced by a negative charge in one of the radicals or by an acid anion,
for dyeing keratin-containing fibres, in particular human hair.

2. Use according to Claim 1, **characterized in that** R⁵ and R²³, independently of one another, are a methyl group, an ethyl group, a hydroxyethyl group or a 2-sulphonatepropyl group.

3. Use according to one of Claims 1 and 2, **characterized in that** R¹⁷, R²⁵ and R²⁶, independently of one another, are hydrogen, a methyl group, an ethyl group, a methoxy group, a phenyl group, an SO₂CF₃ group or a carboxyethyl group.

4. Use according to one of Claims 1 to 3, **characterized in that** one of the compounds specified in Table 1 is used as compounds with the formula I, where the substituents not defined in the table are hydrogen.
**Table 1.1**
| | A | X | R⁵ | B | A' | Y | R²³ | Counterion |
|---|---|---|---|---|---|---|---|---|
| Compound 1 | A1 | S | CH₃ | =CH- | A14 | S | C₂H₅ | iodide |
| Compound 2 | A1 | S | CH₃ | =CH- | A14 | S | 3-sulphonate-propyl | - |
| Compound 3 | A1 | S | CH₃ | =CH- | A12 | S | 3-sulphonate-propyl | - |
| Compound 4 | A1 | S | CH₃ | =CH- | A13 | S | 3-sulphonate-propyl | - |
| Compound 5 | A1 | S | CH₃ | =CH- | A12 | S | C₂H₅ | iodide |
| Compound 6 | A1 | S | CH₃ | =CH- | A13 | S | CH₃ | iodide |

5. Use according to one of Claims 1 to 4, **characterized in that** the agent comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

6. Use according to one of Claims 1 to 5, **characterized in that** the agent comprises direct dyes from the group of nitrophenylenediamines, nitroaminophenols, anthraquinones or indophenols, preferably in an amount of from 0.01 to 20% by weight, based on the total dyeing agent.

7. Use according to one of Claims 1 to 6, **characterized in that** ammonium or metal salts chosen from the group of formates, carbonates, halides, sulphates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc are added.

8. Use according to one of Claims 1 to 7, **characterized in that** the agent comprises oxidizing agents, in particular H₂O₂, in an amount of from 0.01 to 6% by weight, based on the application solution.

9. Use according to one of Claims 1 to 8, **characterized in that** the agent comprises anionic, zwitterionic or nonionic surfactants.

10. Method of dyeing keratin-containing fibres, in particular human hair, in which a dyeing agent comprising at least one compound with the formula I and customary cosmetic ingredients is applied to the keratin-containing fibres, left on the fibres for a certain time, usually about 30 minutes, and then rinsed out again or washed out with a shampoo.

## Revendications

1. Utilisation d'un produit contenant en tant que composant colorant des composés ayant pour formule I,
A=CH=B-CH=A' (I)
dans laquelle =B- représente un groupe =CR¹- ou un groupe 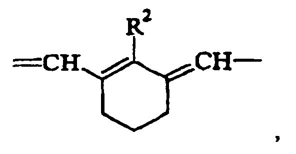 A est choisi parmi les groupes 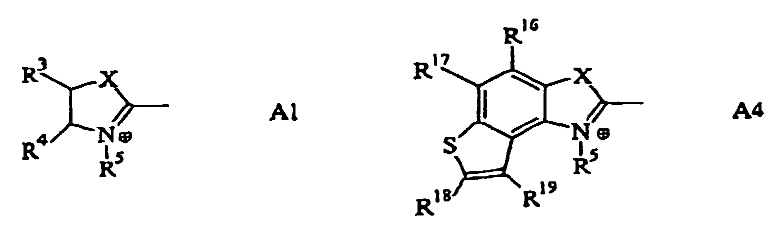 et A' est choisi parmi les groupes 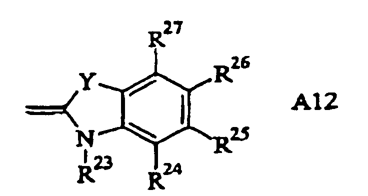 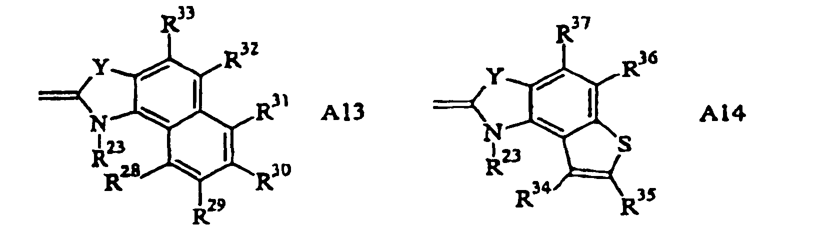 dans lesquels
X et Y représentent respectivement un atome de soufre,
R¹ représente un atome d'hydrogène, ou un groupe NH₂,
R² représente un atome d'hydrogène ou un atome d'halogène, en particulier un atome de chlore,
R⁵ et R²³ représentent indépendamment l'un de l'autre, un groupe alkyle en C₁ à C₄, qui est substitué éventuellement par un ou plusieurs groupes hydroxy ou par un groupe acide ou à anion acide, en particulier un groupe SO₃⁻
R¹⁷, R²⁵ et R²⁶ représentent indépendamment l'un de l'autre un atome d'hydrogène, un groupe alkyle en C₁ à C₄, un groupe alcoxy en C₁ à C₄, un groupe phényle, un groupe SO₂CF₃, ou un groupe carboxy estérifié par un groupe alkyle en C₁ à C₄,
et les autres radicaux représentent indépendamment l'un de l'autre un atome d'hydrogène, un atome d'halogène, un groupe alkyle en C₁ à C₄, ou un groupe alcoxy en C₁ à C₄, qui sont substitués éventuellement par un ou plusieurs groupes hydroxy ou atomes d'halogène,
la charge positive pouvant être compensée par une charge négative dans l'un des radicaux ou bien par un anion acide,
pour colorer les fibres kératiniques, en particulier les cheveux humains.

2. Utilisation selon la revendication 1, **caractérisée en ce que** R⁵ et R²³ représentent indépendamment l'un de l'autre, un groupe méthyle, un groupe éthyle, un groupe hydroxyéthyle, ou un groupe 2-sulfonate-propyle.

3. Utilisation selon l'une quelconque des revendications 1 ou 2, **caractérisée en ce que** R¹⁷, R²⁵ et R²⁶ représentent indépendamment l'un de l'autre un atome d'hydrogène, un groupe méthyle, un groupe éthyle, un groupe méthoxy, un groupe phényle, un groupe SO₂CF₃, ou un groupe carboxyéthyle.

4. Utilisation selon l'une quelconque des revendications 1 à 3, **caractérisée en ce que** l'un des composés cités dans le tableau 1 est mis en oeuvre, en tant que composés de formule I, les substituants non définis dans le tableau représentant un atome d'hydrogène.
**Tableau 1.1**
| | A | X | R⁵ | B | A' | Y | R²³ | Contre-ion |
|---|---|---|---|---|---|---|---|---|
| Composé 1 | A1 | S | CH₃ | =CH- | A¹⁴ | S | C₂H₅ | Iodure |
| Composé 2 | A1 | S | CH₃ | =CH- | A¹⁴ | S | 3-sulfonate-propyle | -- |
| Composé 3 | A1 | S | CH₃ | =CH- | A¹² | S | 3-sulfonate-propyle | -- |
| Composé 4 | A1 | S | CH₃ | =CH- | A¹³ | S | 3-sulfonate-propyle | -- |
| Composé 5 | A1 | S | CH₃ | =CH- | A¹² | S | C₂H₅ | Iodure |
| Composé 6 | A1 | S | CH₃ | =CH- | A¹³ | S | CH₃ | Iodure |

5. Utilisation selon l'une quelconque des revendications 1 à 4, **caractérisée en ce que** le produit contient un agent renforçant les couleurs choisi dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou leurs mélanges quelconques.

6. Utilisation selon l'une quelconque des revendications 1 à 5, **caractérisée en ce que** le produit contient des colorants montant directement sur la fibre, issus du groupe formé par les nitrophénylène-diamines, les nitroaminophénols, les anthraquinones, ou les indophénols, de préférence en une quantité de 0,01 à 20% en poids, par rapport à la teinture totale.

7. Utilisation selon l'une quelconque des revendications 1 à 6, **caractérisée en ce que** des sels d'ammonium ou métalliques sont ajoutés, choisis dans le groupe formé par les formiates, les carbonates, les halogénures, les sulfates, les butyrates, les valériates, les caproates, les acétates, les lactates, les glycolates, les tartrates, les citrates, les gluconates, les propionates, les phosphates et les phosphonates de métaux alcalins, comme le potassium, le sodium ou le lithium, de métaux alcalino-terreux, comme le magnésium, le calcium, le strontium ou le baryum, ou d'aluminium, de manganèse, de fer, de cobalt, de cuivre ou de zinc.

8. Utilisation selon l'une quelconque des revendications 1 à 7, **caractérisée en ce que** le produit contient un agent d'oxydation, en particulier H₂O₂, en une quantité de 0,01 à 6% en poids, par rapport à la solution d'application.

9. Utilisation selon l'une quelconque des revendications 1 à 8, **caractérisée en ce que** le produit contient des tensio-actifs anioniques, zwitterioniques, ou non ioniques.

10. Procédé pour teindre les fibres kératiniques, en particulier les cheveux humains, dans lequel une teinture contenant au moins un composé de formule I ainsi que les ingrédients cosmétiques habituels, est appliquée sur les fibres kératiniques, est laissée sur les fibres un certain temps, habituellement environ 30 minutes, puis elle est de nouveau rincée ou éliminée par lavage avec un shampoing.
